# EUROPÄISCHE PATENTSCHRIFT

(11) **EP 0 705 840 B1**
(45) Veröffentlichungstag und Bekanntmachung des Hinweises auf die Patenterteilung: **09.08.2000**
(21) Anmeldenummer: 95115005.1
(22) Anmeldetag: 23.09.1995
(51) Int. Cl.: C07F 9/40, C07C 69/65

(54) **Verfahren zur Herstellung von 0,0-Dialkyl-4-phosphono-2-methyl-2-butensäurealkylestern und 4-Halogen-2-methyl-2-butensäurealkylestern mit hohem Anteil an E-Isomeren**
Process for the preparation of 0,0-dialkyl-4-phosphono-2-methyl-2-butenic acid alkylesters and 4-halogeno-2-methyl-2-butenic acid alkylesters with high content of E-isomers
Procédé de préparation d'alkylesters de l'acide 0,0-dialkyl-4-phosphono-2-méthyl-2-buténique et d'alkylesters de l'acide 4-halogène-2-méthyl-2-buténique à forte teneur d'isomères E

(30) Priorität: 04.10.1994 DE 4435421
(43) Veröffentlichungstag der Anmeldung: 10.04.1996
(73) Patentinhaber: BASF AKTIENGESELLSCHAFT, 67056 Ludwigshafen (DE)
(72) Erfinder: Krause, Wolfgang, Dr., D-68782 Brühl (DE); Ernst, Hansgeorg, Dr., D-67346 Speyer (DE); Paust, Joachim, Dr., D-67141 Neuhofen (DE); Rheude, Udo, Dr., D-67166 Otterstadt (DE); Dobler, Walter, Dr., D-69126 Heidelberg (DE)

(56) Entgegenhaltungen:
- EP-A- 0 294 774
- DE-B- 2 121 361
- TETRAHEDRON, Bd.44, Nr.15, 1988 Seiten 4713 - 4719 T. KITAHARA ET AL

## Beschreibung

Die Erfindung betrifft ein technisch sehr einfaches und vorteilhaftes Verfahren zur Herstellung von 0,0-Dialkyl-4-phosphono-2-methyl-2-butensäurealkylestern mit hohem Anteil an E-Isomeren (der Einfachheit halber 0,0-Dialkyl-4-phosphono-tiglinsäurealkylester genannt) sowie die vorteilhafte Herstellung von 4-Halogen-2-methyl-2-butensäurealkylestern mit hohem Anteil an E-Isomeren (hier 4-Halogen-tiglinsäureester genannt).

Die 0,0-Dialkyl-4-phosphono-tiglinsäuremethyl- und -ethylester sind begehrte Bausteine für Horner-Emmons-Reaktionen zur Synthese von Polyenen. Sie werden beispielsweise für die Herstellung von den als natürliche Farbstoffe begehrten β-Apo-8'-carotinsäureestern oder β-Apo-4'-carotinsäureestern (vgl. DE-A-3 244 272; CH-A-850 137 oder Helv. Chim. Acta 1959, Seiten 864-70) oder für die Herstellung von Crocetindiestern (vgl. Angew. Chem. 72 (1960), Seiten 911-15) benötigt.

Die 4-Halogen-tiglinsäurealkylester können außer in die 0,0-Dialkyl-4-phosphono-tiglinsäurealkylester z.B. in die entsprechenden 4-Triarylphosphoniumderivate überführt werden, die begehrte C₅-Bausteine für Wittig-Reaktionen sind (vgl. Liebigs Ann. Chem. 1977, Seiten 1146-59, bes. 1150-51).

Eine bekannte Methode zur Darstellung von 4-Brom-2-methyl-butensäureestern ist die Reaktion von Tiglin- oder Angelikasäureestern mit N-Brom-succinimid in halogenierten Kohlenwasserstoffen unter Belichtung (vgl. Beilstein, 4. Ergänzungswerk Bd. 2., Seiten 1555 und Helv. Chim. Acta 1970, Seiten 383 und 394).

Nachteilig an diesem Verfahren sind die dabei erzielten mäßigen Selektivitäten sowie technisch sehr aufwendige Reaktionsbedingungen.

Aus Liebigs Ann. Chem. 1977, Seiten 1146-59 ist ein Verfahren zur Herstellung von 4-Chlor-2-methyl-2-butensäureethylester durch Erhitzen von Vinylmilchsäureethylester mit Thionylchlorid bekannt. Nachteilig an diesem Verfahren sind die mäßigen Ausbeuten, die Anwendung eines großen Überschusses an dem Halogenierungsreagenz sowie die Freisetzung von großen Mengen an SO₂-Gas, die bei technischen Verfahren sehr problematisch ist.

Weiterhin ist aus Tetrahedron 44 (1988), Seiten 4713-20 ein Verfahren zur Herstellung von 0,0-Diethyl-4-phosphono-tiglinsäuremethylester bekannt, bei dem Brenztraubensäure mit Vinylmagnesiumbromid bei Temperaturen von -10 bis -5°C zu Vinylmilchsäure umgesetzt wird, diese mit Diazomethan in den Methylester überführt, dieser nach einer chromatographischen Reinigung an SiO₂ in Gegenwart von Pyridin in Diethylether als Lösungsmittel bei ca. 0°C mit Phosphortribromid in 4-Brom-tiglinsäuremethylester überführt und dieser mit Phosphorigsäuretriethylester in 0,0-Diethyl-4-phosphono-tiglinsäuremethylester überführt wird. Nachteilig an diesem Verfahren sind die Notwendigkeit bei sehr niedrigen Temperaturen zu arbeiten, der Einsatz großer Mengen von Lösungsmitteln sowie die Mitverwendung von giftigem Pyridin bei der Umsetzung mit Phosphortribromid sowie die Notwendigkeit einer SiO₂-Chromatographie zwecks Isolierung von rohem 4-Halogen-tiglinsäureester. Trotz des für ein Verfahren im technischen Maßstab unzumutbaren Aufwandes und der auftretenden Probleme bei der Rückführung von Zusatz- und Hilfsstoffen werden nur Ausbeuten von 35 % d. Theorie, bezogen auf eingesetzte Brenztraubensäure, erhalten.

Aus der EP 294 774, DE-A 3 244 273 und EP-A 110 329 waren ferner Verfahren zur Herstellung der 4-Halogen-2-methyl-2-butensäureestern der allgemeinen Formel III durch Dehydrohalogenierung von 3,4-Dihalogen-butansäureestern bekannt. Nachteilhaft an diesen Verfahren ist die Verwendung von elementarem Brom oder Chlor, die verfahrens- und sicherheitstechnisch aufwendig ist. Zudem werden die 4-Halogen-tiglinsäureester als Isomerengemische mit E/Z-Verhältnissen von nur 1:1 bis 3:1 gewonnen, während für die Synthese der oben genannten β-Apocarotinsäureester, die in all-E-Konfiguration vorliegen, ein möglichst isomerenreiner E-C₅-Baustein benötigt wird.

Aus Houben-Weyl, Methoden der Organischen Chemie, Bd 5/4, S. 361 ff. insbesondere S. 367 f., 387 f. und Bd. 5/3, S. 898 ff. ist ferner bekannt, daß tertiäre Allylalkohole mit Phosphortribromid oder Phosphortrichlorid nur unter mehr oder weniger vollständiger Allylumlagerung in die entsprechenden Allylhalogenide umgelagert werden können, während primäre Allylalkohole glatt zu den entsprechenden Halogeniden reagieren (siehe Helv. Chim. Acta 1966, S. 369-90).

Die Ausbeuten sind im allgemeinen mäßig und erreichen insbesondere bei ungesättigten Allylalkoholen in einigen Fällen ca. 80%, in den meisten Fällen jedoch deutlich weniger (Houben-Weyl, Methoden der Organischen Chemie, Bd 5/4, S. 398-9). Als vorteilhaft wird zudem der Zusatz von tertiären Aminen und insbesondere Pyridin beschrieben.

Es war daher die Aufgabe der Erfindung Verfahren zur Herstellung von 4-Halogen-tiglinsäurealkylestern und 0,0-Dialkyl-4-phosphonotiglinsäurealkylestern zu entwickeln, die diese unter Überwindung der beschriebenen Nachteile des Standes der Technik ausgehend von gut zugänglichen Ausgangsstoffen, in Abwesenheit von giftigen Reaktionshilfsstoffen, wie Pyridin, und auf technisch möglichst einfache Weise in möglichst guten Ausbeuten ergeben.

Es wurde nun überraschenderweise gefunden, daß die als Zwischenprodukt von dem Pflanzenschutzmittel Ronilan® großtechnisch einfach zugänglichen 2-Hydroxy-2-methyl-3-butensäurealkylester (vgl. EP-A-11 855) auch ohne Mitverwendung von Pyridin und vorteilhaft auch ohne Mitverwendung von Lösungsmitteln bei moderaten Temperaturen mit Phosphortribromid oder Phosphortrichlorid in sehr guten Ausbeuten und sehr guten E/Z-Selektivitäten zu 4-Halogen-2-methyl-2-butensäurealkylestern umgesetzt werden können, welche ihrerseits auf technisch einfache Weise mit sehr guten Ausbeuten und sehr guten E/Z-Selektivitäten zu den 0,0-Dialkyl--4-phosphono-2-methyl-2-butensäurealkylestern umgesetzt werden können.

Gegenstand der Erfindung ist dementsprechend ein Verfahren zur Herstellung von 0,0-Dialkyl-4-phosphono-2-methyl-2-butensäurealkylestern der allgemeinen Formel I 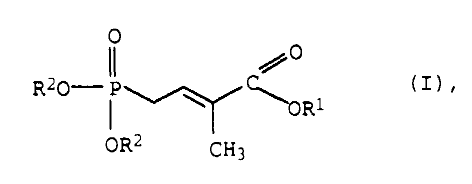 in der R¹ und R² für Methyl oder Ethyl stehen,
mit einem hohem Anteil an E-Isomeren, das dadurch gekennzeichnet ist, daß man
A. den entsprechenden 2-Hydroxy-2-methyl-3-butensäurealkylester der allgemeinen Formel II 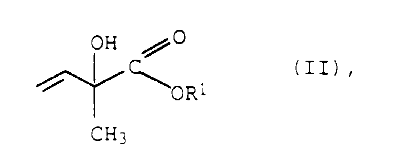 bei Temperaturen von 0 bis 80°C, vorzugsweise 20 bis 70°C, in Abwesenheit von Pyridin und vorzugsweise in Abwesenheit nennenswerter Mengen an Lösungsmitteln mit Phosphortribromid oder Phosphortrichlorid zu einem Gemisch aus einem 4-Halogen-2-methyl-2-butensäureester der allgemeinen Formel III und einem 2-Halogen-2-methyl-3-butensäurealkylester der allgemeinen Formel IV 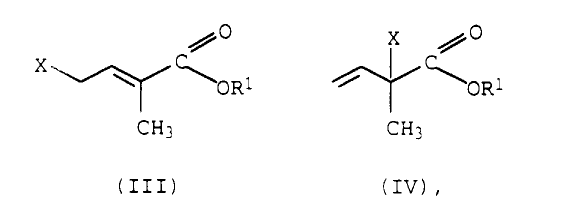 in denen R¹ die oben angegebene Bedeutung hat und X für Chlor oder Brom steht, umsetzt und
B. das erhaltene Gemisch bei Temperaturen von 70 bis 140°C mit Phosphorigsäuretrialkylestern der allgemeinen Formel V 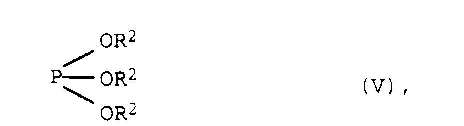 in der R² die oben angegebene Bedeutung hat, umsetzt.
   Gegenstand der Erfindung ist auch ein Verfahren zur Herstellung von 4-Halogen-2-methyl-2-butensäurealkylestern der allgemeinen Formel III 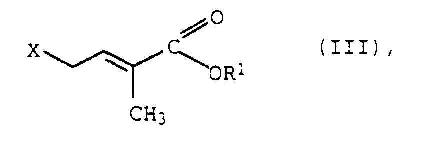 in der
   R¹ für Methyl oder Ethyl steht und
   X Chlor oder Brom bedeutet, mit einem hohen Anteil an E-Isomeren,
   das dadurch gekennzeichnet ist, daß man den entsprechenden 2-Hydroxy-2-methyl-3-butensäurealkylester der allgemeinen Formel II 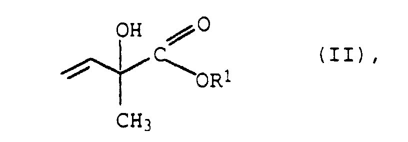 bei Temperaturen von 20 bis 80°C, vorzugsweise 60 bis 80°C, in Abwesenheit von Pyridin und vorzugsweise in Abwesenheit nennenswerter Mengen eines Lösungsmittels mit Phosphortribromid oder Phosphortrichlorid umsetzt.

Für die Überführung dieses Verfahrens in den technischen Maßstab ist von großem Vorteil, daß die Reaktion auch bei moderaten Temperaturen und in Abwesenheit von Lösungsmitteln oder Zusatzstoffen, wie dem giftigen Pyridin, vollständig verläuft, da somit die Abfallbilanz deutlich verbessert werden kann. Als einziges erwähnenswertes Nebenprodukt fällt die unbedenkliche Phosphorigsäure an. Besonders überraschend ist der bei Allylumlagerung sehr hohe Anteil an E-Isomerem der Formel III, der für die Weiterverarbeitung zu Apocarotinsäureestern vorteilhaft ist. Es war weiterhin überraschend, daß auch die als Nebenprodukte gebildeten isomeren 2-Halogen-2-methyl-3-butensäurealkylester der Formel IV bei der anschließenden Umsetzung mit Phosphorigsäuretrialkylestern (Arbusov-Reaktion) in die für Horner-Emmons-Reaktionen begehrten 0,0-Dialkyl-4-phosphono-2-methyl-2-butensäureester mit hohem Anteil an E-Isomeren umgesetzt werden.

Zur Durchführung der Verfahrensstufe A. geht man im allgemeinen so vor, daß man Phosphortribromid bzw. Phosphortricnlorid zu den 2-Hydroxy-2-methyl-3-butensäurealkylestern der allgemeinen Formel II in Abwesenheit von Lösungsmitteln und weiteren Hilfsstoffen so zudosiert, daß maximal Temperaturen von 80°C im Reaktionsgemisch auftreten. Mit Vorteil arbeitet man bei Temperaturen von 20 bis 70°C, bei Verwendung von Phosphortribromid besonders vorteilhaft bei Temperaturen von 20 bis 40°C.

Das molare Verhältnis von 2-Hydroxy-2-methyl-3-butensäurealkylestern der allgemeinen Formel II zu Phosphortrihalogenid beträgt im allgemeinen 3:1 bis 2,6:1, vorzugsweise 2,9:1 bis 2,7:1. Die Zudosierzeiten für die Phosphortrihalogenide betragen 0,5 bis 8, vorzugsweise 1 bis 4 Stunden. Die Zudosierung hängt von den Möglichkeiten zur Abfuhr der Reaktionswärme ab. Dementsprechend sind bei optimaler Gegenkühlung sehr kurze Dosierzeiten möglich.

Die Nachrührzeiten liegen im allgemeinen bei 0 bis 2 Stunden, vorzugsweise 0,5 bis einer Stunde, bei den oben angegebenen Temperaturen.

Die Umsetzung wird im allgemeinen in Abwesenheit "nennenswerter Mengen" von Lösungsmittel durchgeführt. Durch den Ausdruck "nennenswerte Mengen an Lösungsmitteln möchten wir die Gefahr von solchen Ausschlußansprüchen bannen, daß sie durch Zugabe geringer Mengen von Lösungsmitteln, die keinen Einfluß auf die Umsetzung haben können, umgehbar werden. Unter dem Ausdruck nennenswerter Mengen verstehen wir in diesem Sinne Mengen von bis zu etwa 10 Gew.-%, vorzugsweise bis zu 4 Gew.-%.

Im Verfahrensschritt A. erhält man die 4-Halogen-2-methyl-2-butensäurealkylester der Formel III bei Verwendung von Phosphortribromid in Ausbeuten von etwa 90 % bei einem E/Z-Verhältnis von 25:1 bis 40:1 neben zusätzlich etwa 1 bis 5 % an dem tertiären Bromid der Formel IV. Bei Verwendung von Phosphortrichlorid erhält man die 4-Halogen-2-methyl-2-butensäurealkylester der Formel III zwar nur in Ausbeuten von etwa 75 bis 80 % bei einem Isomerenverhältnis von 19:1 bis 25:1, jedoch zusätzlich etwa 10 bis 15 % an den 2-Chlor-2-methyl-3-butensäureestern der Formel IV. Da letztere bei der weiteren Umsetzung auch zu den 0,0-Dialkyl-4-phosphono-2-methyl-2-butensäureestern umgesetzt werden, ergeben sich bei dem erfindungsgemäßen Verfahren Gesamtausbeuten an 0,0-Dialkyl-4-phosphono-2-methyl-2-butensäureestern von bis zu etwa 90 %, bezogen auf eingesetzten 2-Hydroxy-2-methyl-3-butensäurealkylester.

zur Aufarbeitung des Reaktionsgemisches der Reaktionsstufe A. werden die Wertproduktphase und die Phosphorigsäurephase getrennt. Zur vollständigen Gewinnung der Halogenbutensäurealkylester der allgemeinen Formel III und des Isomeren IV wird die Phosphorigsäurephase noch einmal mit einem inerten Lösungsmittel, wie einem Kohlenwasserstoff, vorzugsweise Hexan oder Heptan, extrahiert.

Zur weiteren Umsetzung in die 4-Phosphono-tiglinsäurealkylester der allgemeinen Formel I werden die vereinigten organischen Phasen vorzugsweise bei Temperaturen von 40 bis 50°C und bei Drucken von 2,50·10⁴Pa (250 mbar) bis 1,00·10⁴Pa (100 mbar) eingedampft.

Die so gewonnenen rohen 4-Halogen-2-methyl-2-butensäureester können ohne weitere Reinigung zu 4-Phosphono-2-methyl-2-butensäurealkylestern umgesetzt werden.

Möchte man die 4-Halogen-2-methyl-2-butensäureester für andere Umsetzungen einsetzen und daher möglichst reine 4-Halogen-2-methyl-2-butensäureester und nur geringe Anteile an den 2-Halogen-2-methyl-3-butensäurealkylestern der Formel IV erhalten, ist es ratsam, die Umsetzung des 2-Hydroxy-2-methyl-3-butensäure-alkylesters mit Phosphortribromid oder Phosphortrichlorid bei Temperaturen von 20 bis 80°C, vorzugsweise 60 bis 80°C durchzuführen, insbesondere aber mit Phosphortribromid und bei Temperaturen von 70 bis 80°C durchzuführen.

Zur Durchführung der Verfahrensstufe B. geht man im allgemeinen so vor, daß man die im Reaktionsschritt A. erhaltenen Halogenide bei Temperaturen zwischen 70 und 140°C, vorzugsweise 90 und 120°C, zu den Trialkylphosphiten der allgemeinen Formel V zudosiert, wobei weder Lösungsmittel noch andere Reaktionshilfsstoffe notwendig sind. Die Dosierzeit beträgt etwa 0,5 bis 8, vorzugsweise 1 bis 4 Stunden.

Das Trialkylphosphit verwendet man im allgemeinen in Mengen von 1 bis 1,1 Mol pro Mol eingesetzten 2-Hydroxy-2-methyl-3-butensäureester der Formel II.

i Das bei dieser sogenannten Arbusov-Reaktion anfallende Alkylhalogenid kann durch Abdestillieren aus dem Reaktionsgemisch über eine geeignete Kolonne in Reinheiten von bis zu 99 % gewonnen und weiterverwendet werden.

Die rohen 0,0-Dialkyl-4-phosphono-2-methyl-2-butensäurealkylester werden anschließend durch Abdestillieren von Leicht- und Zwischensiedern bei Temperaturen von 100 bis 140°C und Drücken von 2·10² bis 20.10²Pa (2 bis 20 mbar), vorzugsweise 5·10² bis 15·10² Pa (5 bis 15 mbar), gereinigt.

Auf diese technisch einfache Weise werden die 0,0-Dialkyl-4-phosphono-2-methyl-2-butensäurealkylester der allgemeinen Formel I mit sehr hohen Anteilen an E-Isomeren in Ausbeuten von 85 bis 95 % aus den Halogenverbindungen gewonnen.

Die E/Z-Verhältnisse des Produktes betragen bei Einsatz des 4-Brom-2-methyl-2-butensäuresters 19:1 bis 30:1 bei einer Reinheit von ≥ 95 % und bei Einsatz des 4-Chlor-2-methyl-2-butensäureester 10:1 bis 25:1 bei einer vergleichbaren Reinheit. Eine weitere Reinigung durch Destillation ist nicht notwendig.

Das E/Z-Isomerengleichgewicht von 0,0-Dialkyl-4-phosphono-2-methyl-2-butensäurealkylestern ist temperaturabhängig. So nimmt der unerwünschte Z-Anteil mit steigender Temperatur zu. Bei Erhitzen auf 140°C beträgt das E/Z-Verhältnis 15:1; bei 170°C findet Isomerisierung nach E/Z = 7,4:1 statt. Bei 230°C liegt das Isomerengleichgewicht gar bei 5,4:1. Gemäß oben beschriebener Durchführung der Arbusov-Reaktion wird deutlich, daß Temperaturen von kleiner oder gleich 140°C vorteilhaft für die Gewinnung sehr hoher E-Gehalte sind.

Die erfindungsgemäß erzielten hohen E/Z-Verhältnisse der Verfahrensprodukte demonstrieren den großen Vorteil gegenüber den Verfahren des Standes der Technik. Gemäß dem Verfahren der oben zitierten EP 294 774 werden trotz einer besonderen Temperaturführung während der Umsetzung mit Phosphorigsäuretrialkylestern (Arbusov-Reaktion) die 0,0-Dialkyl-4-phosphono-2-methyl-2-butensäurealkylester nur in einem E/Z-Verhältnis von 7:1 bis maximal 8:1 erhalten und gemäß den in J. Chem. Soc. C 1966, Seiten 2163 f und J. Chem. Soc. C 1968, Seite 1991 f beschriebenen Verfahren werden trotz vergleichbarer Bedingungen bei der Arbusov-Reaktion, die 0,0-Diethyl-4-phosphono-2-methyl-2-butensäuremethylester als Isomerengemisch mit einem E/Z-Verhältnis von lediglich 3:2 erhalten.

Mit Hilfe des erfindungsgemäßen Verfahrens können die begehrten 4-Halogen-2-methyl-2-butensäurealkylester der allgemeinen Formel III in Ausbeuten bis zu 90 % der Theorie als Isomerengemisch mit einem E/Z-Verhältnis von 18:1 bis 40:1 und die 0,0-Dialkyl-4-phosphono-2-methyl-2-butensäurealkylester überraschenderweise in Gesamtausbeuten von bis zu 91 % der Theorie, bezogen auf die eingesetzten 2-Hydroxy-2-methyl-3-butensäurealkylester, als Isomerengemisch mit einem E/Z-Verhältnis von 10:1 bis 30:1 erhalten werden.

### Beispiel 1

a) Herstellung von 4-Brom-tiglinsäureethylester
   2274 g (15,55 mol) eines 98,6%igen Vinylmilchsäureethylesters (VMEE) wurden bei 25°C vorgelegt und in 2 Stunden (h) bei 25°C mit 1540 g (5,69 mol) Phosphortribromid umgesetzt. Es wurde 1 h bei 25°C nachgerührt.
   Zur Hydrolyse überschüssigen Phosphortribromids wurden in 30 Minuten (min) bei 25°C 1,25 Liter (L) Wasser zugegeben. Nach kurzem Nachrühren ließ man absitzen und trennte die untere, das Wertprodukt Bromtiglinsäureethylester enthaltende Phase ab. Auswaage: 3300 g. Die obere Phosphorigsäurephase wurde einmal bei 25°C mit 500 ml technischem Heptan extrahiert. Nach kurzem Rühren wurden die Phasen getrennt. Aus den vereinigten organischen Phasen wurden innerhalb von 1 h bei 45°C und Drucken von 100·10² bis 50·10² Pa (100 bis 50 mbar) Wasserreste ausgekreist sowie das Heptan abdestilliert.
   Die Zusammensetzung des rohen Brom-2-methyl-butensäureestergemisches betrug gemäß Gaschromatographie (GC-Analyse):
   4 % 2-Brom-2-methyl-3-butensäureethylester (Verbindung der Formel IV mit (X = Br und R¹ = Ethyl), 88,8 % 4-Brom-tiglinsäureester der Formel III (X = Br, R¹ = Ethyl), E/Z = 30:1 Auswaage: 3250 g roher Brombutensäureester Ausbeute: 14,56 Mol = 93,7 % (III + IV), bezüglich (bez.) VMEE.
b) Umsetzung des rohen Brom-2-methyl-butensäureestergemisches zu 0,0-Diethyl-4-phosphono-tiglinsäureethylester
   2899 g (17,1 mol) 98%iges Triethylphosphit wurden vorgelegt und auf 120°C erhitzt. Innerhalb von 2 h wurden 3250 g des gemäß 1a hergestellten rohen Brom-2-methyl-butensäureethylestergemisches zugetropft. Die Reaktion begann sofort mit der Zudosierung. Bei der Reaktion gebildetes Ethylbromid (Kₚ = 38°C) wurde laufend über eine Vigreux-Kolonne abdestilliert.
   Nach beendeter Zugabe wurde 30 min bei 120°C nachgerührt. Der rohe 0,0-Diethyl-4-phosphono-2-methyl-2-butensäureethylester wurde bei 120°C bis zum Druck von 15·10² Pa (15 mbar) von Leichtersiedern befreit (Kₚ der Leichtsieder 50 bis 60°C).
   Nach Abdestillation der Leichtersieder wurde der Druck bei 120°C Innentemperatur auf 5 mbar verringert und bei 80°C eine Zwischensiederfraktion abdestilliert. Der nach Abkühlung auf 25°C verbliebene Rückstand von 0,0-Diethyl-4-phosphono-2-methyl-2-butensäureethylester (gelb-oranges Öl) wurde in dieser Qualität als Baustein für Horner-Emmons-Reaktionen zur Herstellung von β-Apo-carotinsäureestern eingesetzt. Ausbeute und Reinheit des 0,0-Diethyl-4-phosphono-2-methyl-2-butensäureethylesters betrugen nach GC-Analyse 3819 g mit einem Gehalt von 96,0 % bei einem E/Z = 25:1 entsprechend einer Ausbeute von 89,3 %, bez. VMEE.

### Beispiel 2

a) Herstellung von 4-Brom-2-methyl-2-butensäureethylester
2274 g (15,55 mol) eines 98,6 %igen VMEE's wurden bei 25°C vorgelegt und in 1,5 h bei 25°C bis maxmial 40°C mit 1540 g (5,69 mol) Phosphortribromid umgesetzt. Es wurde 0,5 h bei 40°C nachgerührt. Zur Hydrolyse des überschüssigen Phosphortribromids wurden in 30 min bei 25°C 1,0 L Wasser zugegeben. Nach kurzem Nachrühren ließ man absitzen und trennte die untere, das Wertprodukt 4-Brom-tiglinsäureethylester enthaltende Phase ab. Auswaage: 3350 g. Die obere Phosphorigsäurephase wurde einmal bei 25°C mit 500 ml technischem Heptan extrahiert. Nach kurzem Rühren wurden die Phasen getrennt.
Aus den vereinigten organischen Phasen wurden bei 50°C und 100·10² bis 50·10² Pa (100 bis 50 mbar) Wasserreste ausgekreist und Heptan abdestilliert.
Zusammensetzung des rohen Brom-2-methyl-butensäureestergemisches betrug gemäß GC-Analyse:
1,9 % 2-Brom-2-methyl-3-butensäureethylester und 89,6 % 4-Brom-tiglinsäuremethylester bei einem E/Z = 21:1.
Auswaage: 3317 g roher Brombutensäureethylester entsprechend einer Ausbeute von 94,3 % (III+IV), bez. VMEE.
b) Herstellung von 0,0-Diethyl-4-phosphono-2-methyl-2-butensäureethylester
2899 g (17,1 mol) 98%iges Triethylphosphit wurden vorgelegt und auf 90°C erhitzt. Innerhalb von 2 h wurden 3317 g des gemäß 2a erhaltenen rohen Brombutensäureethylesters zugetropft. Die Reaktion begann sofort mit der Zudosierung. Bei der Reaktion gebildetes Ethylbromid (Kₚ = 38°C) wurde laufend über eine Vigreux-Kolonne abdestilliert. Nach beendeter Zugabe wurde 1 h bei 90°C nachgerührt.
Der rohe 0,0-Diethyl-4-phosphono-tiglinsäureethylester wurde bei 120°C bis zum Druck von 15·10² Pa (15 mbar) von Leichtersiedern (Kₚ ca. 55°C) befreit.
Nach Abdestillation der Leichtersieder wurde die Temperatur auf 140°C erhöht und der Druck auf 5·10² Pa (5 mbar) verringert und eine Zwischensiederfraktion (Kₚ ca. 75°C) abdestilliert. Der nach Abkühlung auf 25°C verbleibende Rückstand von 0,0-Diethyl-4-phosphono-2-methyl-2-butensäureethylester (gelb-oranges Öl) kann in dieser Qualität bei Horner-Emmons-Reaktionen weiterverarbeitet werden. Ausbeute und Reinheit des Produktes gemäß GC-Analyse:
3862 g mit einem Gehalt von 96,0 % bei einem E/Z = 18:1 entsprechend einer Ausbeute von 90,2 %, bez. VMEE.
Die Abhängigkeit des Isomerengleichgewichtes des 0,0-Diethyl-4-phosphono-2-methyl-2-butensäureethylesters von der Temperatur ist in Tabelle 1 wiedergegeben.

**Tabelle 1**

| T [°C] | Gehalt [Gew.-%] | E/Z-Verhältnis |
|---|---|---|
| 120 | 96,0 | 20:1 |
| 140 | 96,0 | 18:1 |
| 170 | 95,9 | 7,4:1 |
| 200 | 95,7 | 6,3:1 |
| 230 | 95,5 | 5,4:1 |

### Beispiel 3

a) Herstellung von Chlormethylbutensäureethylestern
   365,6 g (2,5 mol) eines 98,6 %igen VMEE's wurden bei 70°C vorgelegt und in 4 h bei 70°C mit 125,7 g (0,915 mol) Phosphortrichlorid umgesetzt. Es wurde 1 h bei 70°C nachgerührt und dann auf 25°C abgekühlt.
   Zur Hydrolyse überschüssigen Phosphortrichlorids wurden in 30 min bei 25°C 200 ml Wasser zugegeben. Nach kurzem Nachrühren ließ man absitzen und trennte die untere Phase mit dem Wertprodukt Chlormethylbutensäureethylester ab.
   Die obere Phosphorigsäurephase wurde einmal bei 25°C mit 80 ml technischem Heptan extrahiert. Nach kurzem Rühren wurden die Phasen getrennt. Aus den vereinigten organischen Phasen wurden bei 45°C und 100·10² bis 50·10² Pa (100 bis 50 mbar) Wasserreste ausgekreist sowie das Heptan weitestgehend abdestilliert.
   Die Zusammensetzung des rohen Chlormethylbutensäureestergemisches betrug gemäß GC-Analyse 12,5 % der Verbindung der Formel IV (X = Cl, R¹ = Et), 75,7 % 4-Chlor-tiglinsäureester III (X = Cl, R¹ = Et) bei E/Z = 21:1.
   Auswaage: 398,3 g roher Chlormethylbutensäureester (88,2%ig) entsprechend einer Ausbeute von 86,4 % (III+IV), bez. VMEE.
b) Herstellung von 0,0-Diethyl-4-phosphono-2-methyl-2-butensäure-ethylester
   466 g (2,75 mol) 98%iges Triethylphosphit wurden vorgelegt und auf 120°C erhitzt. Innerhalb von 4 h wurden 398,3 g des gemäß 3a hergestellten rohen Chlormethylbutensäureethylestergemisches zugetropft. Bei der Reaktion gebildetes Ethylchlorid (Kₚ = 13°C) wurde laufend über eine Vigreux-Kolonne in eine auf -20°C gekühlte Vorlage abdestilliert.
   Nach beendeter Zugabe wurde 2 h bei 90°C nachgerührt. Der rohe 0,0-Diethyl-4-phosphono-2-methyl-2-butensäureethylester wurde bei 120°C bis zum Druck von 15·10² Pa (15 mbar) von Leichtersiedern (Kₚ = ca. 55°C) befreit.
   Nach Abdestillation der Leichtersieder wurde der Druck auf 5 mbar verringert und eine Zwischensiederfraktion (Kₚ = ca. 85°C) abdestilliert.
   Der nach Abkühlung auf 25°C verbleibende Rückstand von 0,0-Diethyl-4-phosphono-tiglinsäureethylester (gelb-oranges Öl) kann in dieser Qualität weiterverarbeitet werden. Ausbeute und Reinheit des 0,0-Diethyl-4-phosphono-tiglinsäureethylesters gemäß GC-Analyse:
   521,4 g mit einem Gehalt von 95,0 % bei einem E/Z = 20:1 entsprechend einer Ausbeute von 75 %, bez. VMEE.

## Patentansprüche

1. Verfahren zur Herstellung von 0,0-Dialkyl-4-phosphono-2-methyl-2-butensäurealkylestern der allgemeinen Formel I 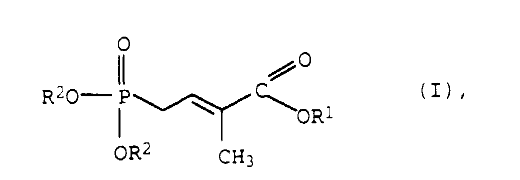 in der R¹ und R² für Methyl oder Ethyl stehen,
mit einem hohem Anteil an E-Isomeren, das dadurch gekennzeichnet ist, daß man
A. den entsprechenden 2-Hydroxy-2-methyl-3-butensäurealkylester der allgemeinen Formel II 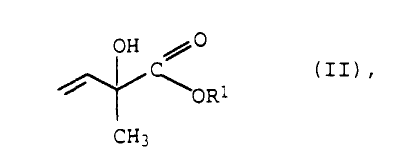 bei Temperaturen von 0 bis 80°C in Abwesenheit von Pyridin mit Phosphortribromid oder Phosphortrichlorid zu einem Gemisch aus einem 4-Halogen-2-methyl-2-butensäureester der allgemeinen Formel III und einem 2-Halogen-2-methyl-3-butensäurealkylester der allgemeinen Formel IV 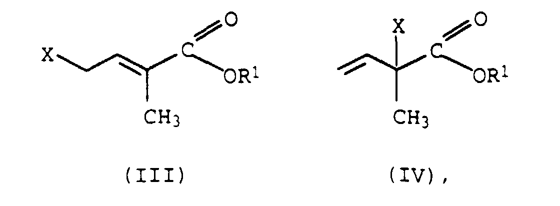 in denen R¹ die oben angegebene Bedeutung hat und X für Chlor oder Brom steht, umsetzt und
B. das erhaltene Gemisch bei Temperaturen von 70 bis 140°C mit Phosphorigsäuretrialkylestern der allgemeinen Formel V 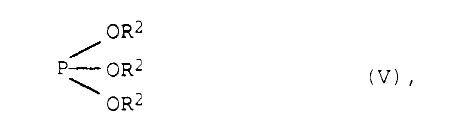 in der R² die oben angegebene Bedeutung hat, umsetzt.

2. Verfahren gemäß Anspruch 1, dadurch gekennzeichnet, daß man im Verfahrensschritt A. die 2-Hydroxy-2-methyl-3-butensäurealkylester der Formel II in Abwesenheit nennenswerter Mengen eines Lösungsmittels mit Phosphortribromid oder Phosphortrichlorid umsetzt.

3. Verfahren gemäß Anspruch 1, dadurch gekennzeichnet, daß man im Verfahrensschritt A. die 2-Hydroxy-2-methyl-3-butensäurealkylester der Formel II bei Temperaturen von 20 bis 70°C mit Phosphortribromid oder Phosphortrichlorid umsetzt.

4. Verfahren gemäß Anspruch 1, dadurch gekennzeichnet, daß man im Verfahrensschritt A. die 2-Hydroxy-2-methyl-3-butensäurealkylester bei Temperaturen von 20 bis 40°C mit Phosphortribromid umsetzt.

5. Verfahren zur Herstellung von 4-Halogen-2-methyl-2-butensäurealkylestern der allgemeinen Formel III 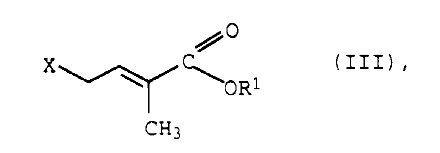 in der
R¹ für Methyl oder Ethyl steht und X Chlor oder Brom bedeutet, mit einem hohen Anteil an E-Isomeren,
dadurch gekennzeichnet, daß man den entsprechenden 2-Hydroxy-2-methyl-3-butensäurealkylester der allgemeinen Formel II 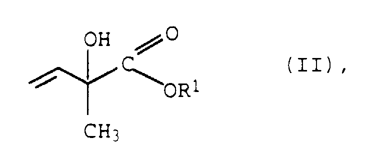 bei Temperaturen von 20 bis 80°C in Abwesenheit von Pyridin mit Phosphortribromid oder Phsphortrichlorid umsetzt.

6. Verfahren gemäß Anspruch 5, dadurch gekennzeichnet, daß man den 2-Hydroxy-2-methyl-3-butensäurealkylester der Formel II in Abwesenheit nennenswerter Mengen eines Lösungsmittels mit Phosphortribromid oder Phosphortrichlorid umsetzt.

7. Verfahren gemäß Anspruch 5, dadurch gekennzeichnet, daß man den 2-Hydroxy-2-methyl-3-butensäurealkylester der Formel II bei Temperaturen von 60 bis 80°C mit Phosphortribromid oder Phosphortrichlorid umsetzt.

8. Verfahren gemäß Anspruch 5, dadurch gekennzeichnet, daß man den 2-Hydroxy-2-methyl-3-butensäurealkylester der Formel II bei Temperaturen von 70 bis 80°C mit Phosphortribromid umsetzt.

## Claims

1. A process for preparing O,O-dialkyl-4-phosphono-2-methyl-2-butenoic acid alkyl esters of the general formula I 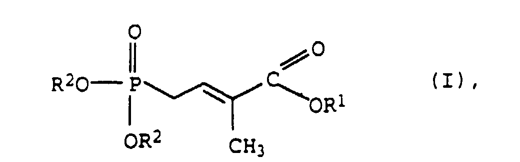 in which R¹ and R² are methyl or ethyl, with a high proportion of E isomers, which comprises
A. reacting the corresponding 2-hydroxy-2-methyl-3-butenoic acid alkyl ester of the general formula II 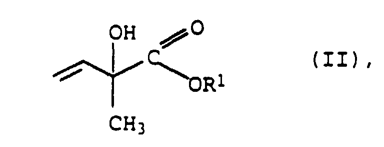 at temperatures of from 0 to 80°C in the absence of pyridine with phosphorus tribromide or phosphorus trichloride to give a mixture of a 4-halo-2-methyl-2-butenoic ester of the general formula III and a 2-halo-2-methyl-3-butenoic acid alkyl ester of the general formula IV 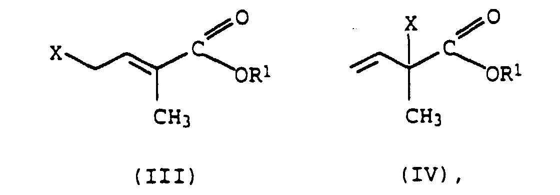 in which R¹ has the abovementioned meaning, and X is chlorine or bromine, and
B. reacting the resulting mixture at temperatures of from 70 to 140°C with trialkyl phosphites of the general formula V 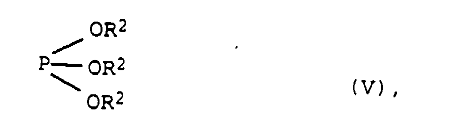 in which R² has the abovementioned meaning.

2. A process as claimed in claim 1, wherein in step A. the 2-hydroxy-2-methyl-3-butenoic acid alkyl ester of the formula II is reacted with phosphorus tribromide or phosphorus trichloride in the presence of negligible amounts of a solvent.

3. A process as claimed in claim 1, wherein in step A. the 2-hydroxy-2-methyl-3-butenoic acid alkyl ester of the formula II is reacted with phosphorus tribromide or phosphorus trichloride at temperatures of from 20 to 70°C.

4. A process as claimed in claim 1, wherein in step A. the 2-hydroxy-2-methyl-3-butenoic acid alkyl ester is reacted with phosphorus tribromide at temperatures of from 20 to 40°C.

5. A process for preparing 4-halo-2-methyl-2-butenoic acid alkyl esters of the general formula III 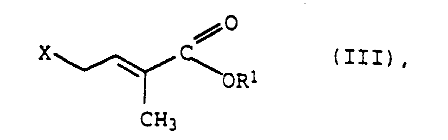 in which R¹ is methyl or ethyl and X is chlorine or bromine, with a high proportion of E isomers, which comprises reacting the corresponding 2-hydroxy-2-methyl-3-butenoic acid alkyl ester of the general formula II 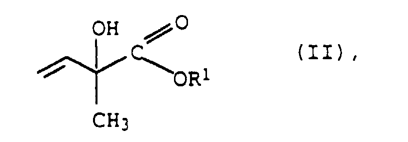 with phosphorus tribromide or phosphorus trichloride in the absence of pyridine at temperatures of from 20 to 80°C.

6. A process as claimed in claim 5, wherein the 2-hydroxy-2-methyl-3-butenoic acid alkyl ester of the formula II is reacted with phosphorus tribromide or phosphorus trichloride in the presence of negligible amounts of a solvent.

7. A process as claimed in claim 5, wherein the 2-hydroxy-2-methyl-3-butenoic acid alkyl ester of the formula II is reacted with phosphorus tribromide or phosphorus trichloride at temperatures of from 60 to 80°C.

8. A process as claimed in claim 5, wherein the 2-hydroxy-2-methyl-3-butenoic acid alkyl ester of the formula II is reacted with phosphorus tribromide at temperatures of from 70 to 80°C.

## Revendications

1. Procédé pour la préparation de O,O-dialkyl-4-phosphono-2-méthyl-2-buténoates d'alkyle de formule générale I 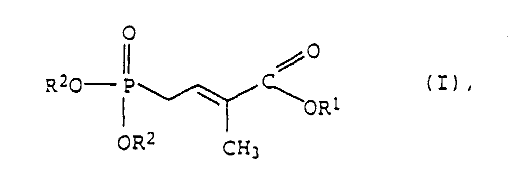 dans laquelle R¹ et R² représentent des groupes méthyle ou éthyle,
à fortes proportions d'isomère E, ce procédé se caractérisant par le fait que
A. on fait réagir les 2-hydroxy-2-méthyl-3-buténoates d'alkyle correspondants de formule générale II 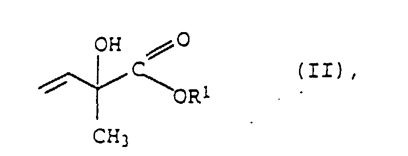 à des températures de 0 à 80°C, en l'absence de pyridine, avec le tribromure de phosphore ou le trichlorure de phosphore, la réaction donnant un mélange d'un ester 4-halogéno-2-méthyl-2-buténoïque de formule III et d'un 2-halogéno-2-méthyl-3-buténoate d'alkyle de formule générale IV 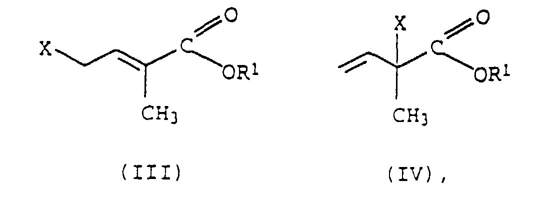 dans lesquelles R¹ a les significations indiquées ci-dessus et X représente le chlore ou le brome, et
B. on fait réagir le mélange obtenu, à des températures de 70 à 140°C, avec des phosphites de trialkyle de formule générale V 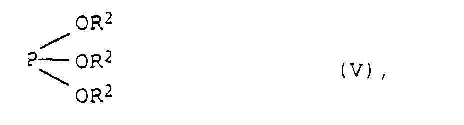 dans laquelle R² a les significations indiquées ci-dessus.

2. Procédé selon la revendication 1, caractérisé par le fait que, au stade opératoire A., on fait réagir les 2-hydroxy-2-méthyl-3-buténoates d'alkyle de formule II avec le tribromure de phosphore ou le trichlorure de phosphore en l'absence de quantités notables d'un solvant.

3. Procédé selon la revendication 1, caractérisé par le fait que, au stade opératoire A., on fait réagir les 2-hydroxy-2-méthyl-3-buténoates d'alkyle de formule II avec le tribromure de phosphore ou le trichlorure de phosphore à des températures de 20 à 70°C.

4. Procédé selon la revendication 1, caractérisé par le fait que, au stade opératoire A., on fait réagir les 2-hydroxy-2-méthyl-3-buténoates d'alkyle avec le tribromure de phosphore à des températures de 20 à 40°C.

5. Procédé pour la préparation de 4-halogéno-2-méthyl-buténoates d'alkyle de formule générale III 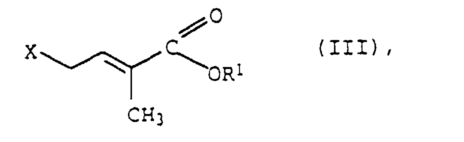 dans laquelle
R¹ représente un groupe méthyle ou éthyle et X le chlore ou le brome, à fortes proportions d'isomère E,
caractérisé par le fait que l'on fait réagir les 2-hydroxy-2-méthyl-3-buténoates d'alkyle correspondants, de formule générale II 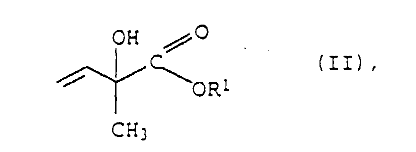 avec le tribromure de phosphore ou le trichlorure de phosphore à des températures de 20 à 80°C et en l'absence de pyridine.

6. Procédé selon la revendication 5, caractérisé par le fait que l'on fait réagir les 2-hydroxy-2-méthyl-3-buténoates d'alkyle de formule II avec le tribromure de phosphore ou le trichlorure de phosphore en l'absence de quantités notables d'un solvant.

7. Procédé selon la revendication 5, caractérisé par le fait que l'on fait réagir les 2-hydroxy-2-méthyl-3-buténoates d'alkyle de formule II avec le tribromure de phosphore ou le trichlorure de phosphore à des températures de 60 à 80°C.

8. Procédé selon la revendication 5, caractérisé par le fait que l'on fait réagir les 2-hydroxy-2-méthyl-3-buténoates d'alkyle de formule II avec le tribromure de phosphore à des températures de 70 à 80°C.
